# EUROPEAN PATENT SPECIFICATION

(11) **EP 3 966 568 B1**
(45) Date of publication and mention of the grant of the patent: **03.07.2024**
(21) Application number: 20801737.6
(22) Date of filing: 05.05.2020
(51) Int. Cl.: G01N 33/573, C07K 16/40, C12Q 1/48, C12Q 1/689, C12N 9/12, G01N 33/569

(54) **RESPIRATORY INFECTION DETECTION AND CLASSIFICATION**
NACHWEIS UND KLASSIFIZIERUNG VON ATEMWEGSINFEKTIONEN
DÉTECTION ET CLASSIFICATION D'UNE INFECTION RESPIRATOIRE

(30) Priority: 06.05.2019 US 201962843652 P
(43) Date of publication of application: 16.03.2022
(73) Proprietor: Arocell AB, 754 50 Uppsala (SE)
(72) Inventor: ERIKSSON, Staffan, 181 46 Lidingö (SE); VENGE, Per, 753 12 Uppsala (SE)
(74) Representative: Barker Brettell Sweden AB
(86) International application number: PCT/SE2020/050448
(87) International publication number: WO 2020/226553

(56) References cited:
- EP-A2- 0 094 923
- WO-A1-2013/114064
- WO-A1-2015/094106
- WO-A1-2018/029690
- WO-A2-2006/002142
- CN-A- 104 672 331
- US-A1- 2004 058 426
- WELIN MARTIN ET AL: "Structures of thymidine kinase 1 of human and mycoplasmic origin", PROCEEDINGS OF THE NATIONAL ACADEMY OF SCIENCES, NATIONAL ACADEMY OF SCIENCES, vol. 101, no. 52, 28 December 2004 (2004-12-28), pages 17970-17975, XP002517209, ISSN: 0027-8424, DOI: 10.1073/PNAS.0406332102
- VENGE PER ET AL: "Human Neutrophil Lipocalin as a Superior Diagnostic Means To Distinguish between Acute Bacterial and Viral Infections", CLINICAL AND VACCINE IMMUNOLOGY, vol. 22, no. 9, 1 July 2015 (2015-07-01), pages 1025-1032, XP055951816, ISSN: 1556-6811, DOI: 10.1128/CVI.00347-15 Retrieved from the Internet: URL:https://www.ncbi.nlm.nih.gov/pmc/artic les/PMC4550662/pdf/zcd1025.pdf>
- VENGE P. et al.: "Human Neutrophil Lipocalin in Activated Whole Blood Is a Specific and Rapid Diagnostic Biomarker of Bacterial Infections in the Respiratory Tract", Clin Vaccine Immunol, vol. 24, 2017, pages 1-9, XP055686429, DOI: 10.1128/CVI.00064-17

## Description

### TECHNICAL FIELD

The present embodiments generally relate to respiratory infections, and in particular to detection and classification of such respiratory infections.

### BACKGROUND

The medically most important mycoplasma infection is a lower tract respiratory infection caused by *Mycoplasma pneumoniae,* which is a very small wall-less bacteria of the class Mollicutes. This disease is presented as an atypical bacterial pneumonia, referred to as *Mycoplasma* pneumonia, with a relatively long incubation period and a wide spectrum of clinical symptoms, but typically with a low intensive persistent dry cough. It is estimated that in the United States about two million cases occur each year and *M. pneumoniae* infections accounts for 1-10 out of every 50 cases of community-acquired pneumonia. It is therefore generally considered as a major health problem worldwide.

Current available diagnostic tests for *M*. *pneumoniae* infections include culturing and serologic methods and polymerase chain reaction (PCR) based methods, and only a limited number of molecular methods are currently in use. Therefore, better diagnostic procedures are much needed.

### SUMMARY

It is a general objective to provide an improved detection and classification of respiratory infections.

This and other objectives are met by embodiments as disclosed herein.

The present invention is defined in the independent claims. Further embodiments of the invention are defined in the dependent claims.

The present embodiments involve using determined level of serum thymidine kinase 1 (STK1) material in a serum or plasma sample to predict presence of and diagnose *Mycoplasma* pneumonia caused by *M. pneumoniae* in a patient. The determined level of STK1 material can also be used to classify a respiratory infection in a patient as either being *Mycoplasma* pneumonia caused by *M. pneumoniae* or one of a viral pneumonia caused by a virus or a bacterial pneumonia caused by a bacterium other than *M. pneumoniae.*

The present embodiments thereby provides a technology to detect and classify the otherwise hard-to-diagnose *M. pneumoniae* infections.

### BRIEF DESCRIPTION OF THE DRAWINGS

The embodiments, together with further objects and advantages thereof, may best be understood by making reference to the following description taken together with the accompanying drawings, in which:
Fig. 1 illustrates serum thymidine kinase 1 (STK1) protein concentrations in healthy subjects (n=144), and persons with acute bacterial (n=98), viral (n=64) or *Mycoplasma pneumoniae* (n=25) infections. The Y-axis shows the 10-log values for the STK1 concentrations and the significance P values are for the pairwise comparisons between healthy and the different patient groups.
Fig. 2 illustrates STK1 protein concentrations in healthy subjects (n=144), and persons with acute bacterial (n=60) or *M*. *pneumoniae* (n=25) lower respiratory tract infections. The Y-axis shows the 10-log values for the STK1 concentrations and the significance P values are for the pairwise comparisons between healthy and the different patient groups.
Fig. 3 shows the distinction between healthy subjects and those with *M. pneumoniae* acute infections. The results from patients with clinical diagnosis and likely *M*. *pneumoniae* etiology of their infections are shown. P values given in the figures are statistical differences between the receiver operating characteristic (ROC) curves of STK1 concentrations.
Fig. 4 illustrates the results of a ROC analysis of the distinction between bacterial and *M. pneumoniae* causes of lower respiratory tract infections.
Fig. 5 illustrates the results of a ROC analysis of the distinction between viral and *M. pneumoniae* causes of lower respiratory tract infections.
Fig. 6 illustrates the results of a ROC analysis of the distinction between bacterial and *M. pneumoniae* causes of lower tract respiratory tract infections.
Fig. 7 illustrates the results of a ROC analysis of the distinction between bacterial and *M. pneumoniae* causes of lower tract respiratory infections in comparison with CRP (C-reactive protein), PCT (procalcitonin) and blood neutrophil counts.
Fig. 8 illustrates the results of a ROC analysis of the distinction between bacterial and *M. pneumoniae* causes of lower tract respiratory infections for serum TK1 (S-TK1), plasma HNL (P-HNL dimer), plasma C-reactive protein (P-CRP), serum procalcitonin (S-PCT) and a ratio between serum TK1 and plasma HNL (TK1/HNL dimer).
Figs. 9A and 9B illustrate serum TK1 (S-TK1) (9A) and plasma HNL (P-HNL dimer) protein concentrations in healthy subjects (n=144), and persons with bacterial pneumonia (n=86), or *Mycoplasma* pneumonia (n=28). The Y-axis shows the 10-log values for the concentrations.

### DETAILED DESCRIPTION

The present embodiments generally relate to respiratory infections, and in particular to detection and classification of such respiratory infections.

The present invention is based on experimental results indicating that respiratory infection caused by *Mycoplasma pneumoniae,* in particular *Mycoplasma* pneumonia, leads to significantly increased levels of thymidine kinase 1 (TK1) protein, in particular serum TK1 (STK1 or S-TK1) protein, in human patients. Such an increase in STK1 protein levels is, however, not seen in healthy humans nor in patients suffering from respiratory infections caused by other pathogens, e.g., viral pneumonia caused by viruses and bacterial pneumonia caused by bacteria other than *M. pneumoniae.*

Hence, TK1 protein levels, such as STK1 protein levels, can thereby be used to identify or diagnose patients with respiratory infections as suffering from *Mycoplasma* pneumonia caused by *M. pneumoniae.* These *Mycoplasma* pneumonia patients can thereby be separated from other patients with respiratory infections caused by pathogens other than *M. pneumoniae.*

This means that TK1 protein determination can be used as a valuable biomarker for the otherwise difficult-to-diagnose *M. pneumoniae* infection, optionally together with other biomarkers, such as C-reactive protein (CRP), procalcitonin (PCT), calprotectin and/or human neutrophil lipocalin (HNL), such as HNL in blood (B-HNL) and/or plasma (P-HNL).

In an embodiment, TK1 is used as a biomarker together with at least one biomarker selected from the group consisting of CRP, PCT, calprotectin and HNL. In a preferred embodiment, TK1 is used as a biomarker together with at least one biomarker selected from the group consisting of CRP, PCT and HNL, more preferably CRP, PCT and P-HNL.

Hence, in an embodiment, TK1 is used together with CRP. In another embodiment, TK1 is used together with PCT and in a further embodiment TK1 is used together with HNL, such as B-HNL and/or P-HNL, preferably P-HNL. In yet another embodiment, TK1 is used together with calprotectin. TK1 may also be used together with at least two additional biomarkers, such as TK1 and CRP, TK1 and PCT, TK1 and calprotectin, or TK1 and HNL, such as B-HNL and/or P-HNL, preferably P-HNL. In further embodiments, TK1 is used together with at least three additional biomarkers, such as TK1, CRP and PCT; TK1, CRP and calprotectin; TK1, CRP and HNL, such as B-HNL and/or P-HNL, preferably P-HNL; TK1, PCT and calprotectin; TK1, PCT and HNL, such as B-HNL and/or P-HNL, preferably P-HNL; or TK1, calprotectin and HNL, such as B-HNL and/or P-HNL, preferably P-HNL. In yet other embodiments, TK1 is used together with at least four additional biomarkers, such as TK1, CRP, PCT and calprotectin, TK1, CRP, PCT and HNL, such as B-HNL and/or P-HNL, preferably P-HNL; TK1, CRP, calprotectin and HNL, such as B-HNL and/or P-HNL, preferably P-HNL; TK1, PCT, calprotectin and HNL, such as B-HNL and/or P-HNL, preferably P-HNL. In another embodiment, TK1 is used together with CRP, PCT, calprotectin and HNL, such as B-HNL and/or P-HNL, preferably P-HNL.

For instance, when TK1 is used together with at least one additional biomarker, the level of TK1 and the level(s) of the at least one additional biomarker is compared to a respective threshold value and then the estimation, diagnosis or classification is based on the comparisons of each biomarker (TK1 and at least one additional biomarker) with its respective threshold value.

In an alternative embodiment, value is calculated based on the level of TK1 and the level(s) of the at least one additional biomarker and that value is compared to a threshold value. For instance, the value could be calculated based on a function f( ), f(TK1, (CRP, PCT, calprotectin and/or HNL)), wherein TK1, CRP, PCT, calprotectin and HNL denotes the respective level or amount determined for the particular biomarker. A typical example of function f( ) that can be used is a ratio or quotient between the levels of two biomarkers, such as TK1/HNL, for instance TK1/P-HNL, or HNL/TK1, such as P-HNL/TK1. Another example of function f( ) is the product of the levels of two or more biomarkers.

TK1 has been used as a serum biomarker for prognosis and monitoring of hematologic malignancies for many years. TK1 is enzyme that phosphorylates thymidine (dThd) to deoxythymidine monophosphate (dTMP) using adenosine triphosphate (ATP) as the phosphate donor. dTMP undergoes further phosphorylation and forms deoxythymidine triphosphate (dTTP), which is incorporated into deoxyribonucleic acid (DNA) during the S phase of the cell cycle.

STK1 has been determined by either enzyme activity assays or more recently with immunoassays.

Clinical studies using a radioactive TK1 enzyme activity assay (TK-REA) demonstrated a prognostic value in serum TK1 measurements in several malignancies, e.g., non-Hodgkin lymphoma, Hodgkin's disease and various forms of leukemia.

In an early study with the TK-REA several different types of malignancies as well as other clinical conditions including viral infections, mononucleosis and *M. pneumoniae* were investigated [1]. It was found that in patients with mononucleosis the TK1 levels were drastically increased in all patients with acute symptoms of disease. In case of TK1 levels in sera from patients sent for virus diagnosis 83 % had lower levels than the cut off levels for healthy persons, while 17 % of the sera showed increased TK1 activity levels. The presence of elevated TK1 activity was associated with rubella, measles, cytomegalovirus and in some cases of herpes simplex virus (HSV) or varicella zoster virus (VZV) infections. However, there was no increase in the TK1 activity levels in subjects with *M. pneumoniae* infection reported [1]. Elevated serum TK1 activities have also been described in patients with viral hepatitis, particularly in hepatitis A infections, and there was significant correlation with aspartate aminotransferase (ASAT), alanine aminotransferase (ALAT) and lactate dehydrogenase (LDH) but not to other liver blood biomarkers.

TK1 activities in sera from patients with acute bacterial infections are not much studied and there are only a few publications on this subject. TK1 activities in sera from 100 healthy subjects were determined in [2, 3] and the mean value was 4 U/L, while in 100 patients with undefined bacterial infections the median value was 10 U/L, similar to what was found in 40 women with benign breast cancer. Correspondingly, 100 patients with undefined viral infections had a mean value of 54 U/L [2, 3].

An aspect of the embodiments relates to a method of predicting the presence of *Mycoplasma* pneumonia in a patient or subject suffering from a respiratory infection. The method comprises determining a level of TK1 material in a body sample obtained from the patient or subject with an anti-TK1 antibody or a fragment thereof. The method also comprises estimating a likelihood that the respiratory infection is *Mycoplasma* pneumonia caused by *M. pneumoniae* based on the level of TK1 material in the body sample.

In an embodiment, the method also comprises determining a level of at least one additional biomarker in the body sample or another body sample obtained from the subject. In this embodiment, the at least one additional biomarker is selected from the group consisting of CRP, PCT, calprotectin and HNL. In this embodiment, estimating the likelihood comprises estimating the likelihood that the respiratory infection is *Mycoplasma* pneumonia caused by *Mycoplasma pneumoniae* based on the determined level of TK1 material in the body sample and based on the determined level of the at least one additional biomarker in the body sample or the another body sample.

Another aspect of the embodiments relates to a method of diagnosing *Mycoplasma* pneumonia in a patient or subject. The method comprises determining a level of TK1 material in a body sample obtained from the patient or subject with an anti-TK1 antibody or a fragment thereof. The method also comprises diagnosing the patient or subject with *Mycoplasma* pneumonia based on the level of TK1 material in the body sample.

In an embodiment, the method also comprises determining a level of at least one additional biomarker in the body sample or another body sample obtained from the subject. In this embodiment, the at least one additional biomarker is selected from the group consisting of CRP, PCT, calprotectin and HNL. In this embodiment, diagnosing the subject comprises diagnosing the subject with *Mycoplasma* pneumonia based on the determined level of TK1 material in the body sample and based on the determined level of the at least one additional biomarker in the body sample or the another body sample.

In an embodiment, the estimating and diagnosing steps in the above presented embodiments are performed based on a comparison between the determined level of TK1 material and a threshold value. In a particular embodiment, the estimating step involves estimating a high likelihood that the respiratory infection is *Mycoplasma* pneumonia caused by *M. pneumoniae* if the determined level of TK1 material is above the threshold value and otherwise estimating a low likelihood that the respiratory infection is *Mycoplasma* pneumonia caused by *M. pneumoniae.* Hence, in an embodiment, if the determined level of TK1 material does not exceed the threshold value, the method comprises estimating a high likelihood that the respiratory infection is caused by a pathogen other than *M. pneumoniae,* such as a viral pneumonia caused by a virus or a bacterial pneumonia caused by a bacterium other than M. *pneumoniae.*

A further aspect of the embodiments relates to a method of classifying a respiratory infection in a subject. The method comprises determining a level of TK1 material in a body sample obtained from the subject with an anti-TK1 antibody or a fragment thereof. The method also comprises classifying the respiratory infection as being a *Mycoplasma* pneumonia caused by *Mycoplasma pneumoniae* or classifying the respiratory infection as being a viral pneumonia caused by a virus or a bacterial pneumonia caused by a bacterium other than *M. pneumoniae* based on the determined level of TK1 material in the body sample.

In an embodiment, the method also comprises determining a level of at least one additional biomarker in the body sample or another body sample obtained from the subject. In this embodiment, the at least one additional biomarker is selected from the group consisting of CRP, PCT, calprotectin and HNL. In this embodiment, classifying the respiratory infection comprises classifying the respiratory infection as being a *Mycoplasma* pneumonia caused by *Mycoplasma pneumoniae* or classifying the respiratory infection as being a viral pneumonia caused by a virus or a bacterial pneumonia caused by a bacterium other than *M. pneumoniae* based on the determined level of TK1 material in the body sample and based on the determined level of the at least one additional biomarker in the body sample or the another body sample.

In a particular embodiment, the method comprises determining a level of HNL in the body sample or another body sample obtained from the subject. The method also comprises calculating a ratio or quotient between the determined level of TK1 material in the body sample and the determined level of HNL in the body sample or the another body sample. In this particular embodiment, classifying the respiratory infection comprises classifying the respiratory infection as being a *Mycoplasma* pneumonia caused by *M. pneumoniae* if the quotient or ratio exceeds a threshold value and otherwise classifying the respiratory infection as being a bacterial pneumonia caused by the bacterium other than *M*. *pneumoniae.*

A further aspect of the embodiments relates to a method of classifying a respiratory infection in a patient or subject. The method comprises determining a level of TK1 material in a body sample obtained from the patient or subject with an anti-TK1 antibody or a fragment thereof. The method also comprises classifying the respiratory infection as being a *Mycoplasma* pneumonia caused by *Mycoplasma pneumoniae* if the level of TK1 material in the body sample exceeds a threshold value and otherwise classifying the respiratory infection as being a viral pneumonia caused by a virus or a bacterial pneumonia caused by a bacterium other than *Mycoplasma pneumoniae.*

Viral pneumonia is a pneumonia caused by a virus. Viruses are the most common cause of pneumonia in children, while in adults bacteria are a more common cause. Common causes of viral pneumonia include influenza virus A and B, respiratory syncytial virus (RSV), human parainfluenza viruses, respiratory syncytial virus, rhinovirus, human bocavirus, and parainfluenza viruses. Rarer viruses that commonly result in pneumonia include adenoviruses, metapneumovirus, severe acute respiratory syndrome (SARS) virus, and Middle East respiratory syndrome (MERS) virus. Viruses that primarily cause other diseases, but sometimes cause pneumonia include herpes simplex virus (HSV), varicella-zoster virus (VZV), measles virus, rubella virus, cytomegalovirus (CMV), smallpox virus and dengue virus

Bacterial pneumonia is a type of pneumonia caused by bacterial infection. Bacterial pneumonia can be caused by both Gram-positive bacteria and Gram-negative bacteria. *Streptococcus pneumoniae* is the most common bacterial cause of pneumonia in all age groups except newborn infants. *S*. *pneumoniae* is a Gram-positive bacterium that often lives in the throat of people who do not have pneumonia. Other important Gram-positive causes of pneumonia are *Staphylococcus aureus* and *Bacillus anthracis.* Gram-negative bacteria are seen less frequently as the cause of pneumonia. *Haemophilus influenzae, Klebsiella pneumoniae, Escherichia coli, Pseudomonas aeruginosa, Bordetella pertussis,* and *Moraxella catarrhalis* are the most common Gram-negative causes of pneumonia. Bacterial pneumonia may also be caused by atypical bacteria, such as *Coxiella burnetii, Chlamydophila pneumoniae,* and *Legionella pneumophila.*

Bacterial pneumonia as used herein means a pneumonia caused by a bacterium other than *M*. *pneumoniae.*

*Mycoplasma* pneumonia is a form of pneumonia caused by *M. pneumoniae. M. pneumoniae* is spread through respiratory droplet transmission. Once attached to the mucosa of a host organism, *M*. *pneumoniae* extracts nutrients, grows, and reproduces by binary fission. Attachment sites include the upper and lower respiratory tract, causing pharyngitis, bronchitis, and pneumonia. The infection caused by this bacterium is sometimes called atypical pneumonia because of its protracted course and lack of sputum production and wealth of extrapulmonary symptoms. *Mycoplasma* pneumonia can be complicated by Stevens-Johnson syndrome, autoimmune hemolytic anemia, cardiovascular diseases, encephalitis, or Guillain-Barre syndrome.

In an embodiment, classifying the respiratory infection comprises classifying the respiratory infection as being a *Mycoplasma* pneumonia caused by *M*. *pneumoniae* if the determined level of TK1 material in the body sample exceeds the threshold value and otherwise classifying the respiratory infection as being a bacterial pneumonia caused by the bacterium other than *M. pneumoniae.*

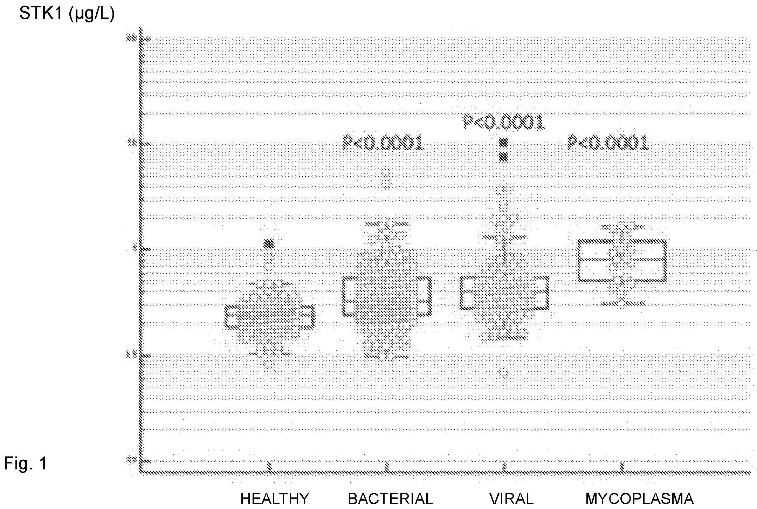

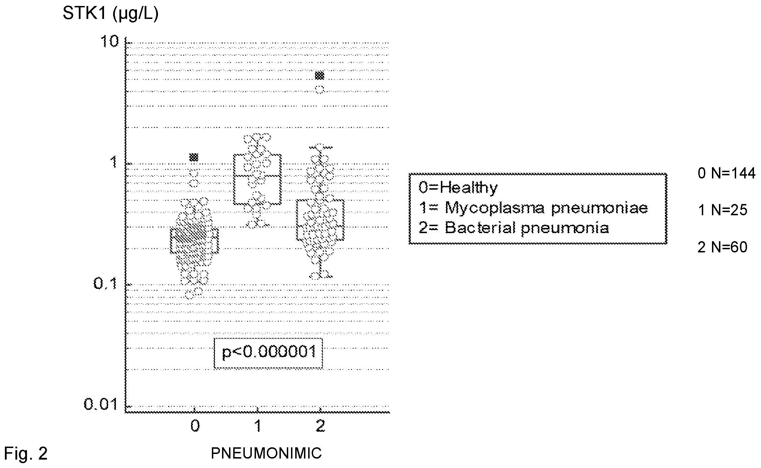

The threshold value used in the method is typically dependent on the method used to measure TK1 in the body sample, such as based on the particular anti-TK1 antibody or antibodies used in the measurements. The threshold value can be determined as disclosed in the Example section by determining the level of TK1 in body samples collected from patients diagnosed with *Mycoplasma* pneumonia, and optionally in body samples collected from healthy subjects and/or patients diagnosed with viral pneumonia or bacterial pneumonia other than *Mycoplasma* pneumonia. The threshold value could then be determined from such measurements. For instance, Figs. 1 and 2 illustrate determined levels of serum TK1 (STK1) material in healthy volunteers and different patient groups using a TK1 Enzyme-Linked Immunosorbent Assay (ELISA) (AroCell TK 210 ELISA). In such a case, a suitable threshold value could be selected within a range of from 0.5 to 1.0 µg/L, such as from 0.6 to 1.0 µg/L, preferably from 0.7 to .9 µg/L, such as 0.7 µg/L or 0.8 µg/L.

In an embodiment, the method also comprises obtaining the body sample from the patient.

The body sample is preferably a body fluid sample comprising TK1 material. Non-limiting, but preferred, examples of such body fluid samples include a serum sample, a plasma sample, a blood sample, a synovial fluid sample, a lymphatic fluid sample, a urine sample and a saliva sample, preferably a serum sample, a plasma sample or a blood sample, and more preferably a serum sample or a plasma sample, such as a lithium-heparin plasma sample.

According to the invention, determining the level of TK1 material comprises determining a level of STK1 material in a serum or plasma sample obtained from the patient with the anti-TK1 antibody or the fragment thereof.

TK1 in humans are present in various forms depending on the presence of certain molecules, e.g., presence or absence of adenosine triphosphate (ATP); depending on the concentration of the protein, i.e., high or low concentration; depending on the type of the protein, i.e., native or recombinant TK1; and depending on the site of the protein, i.e., in serum or cytoplasma.

Generally, cytosolic and recombinant human TK1 occurs as tetramers in the presence of ATP or at high concentration, and as dimers in the absence of ATP or at low concentration. The tetramer form of cytosolic and recombinant human TK1 has high TK1 activity whereas the dimer form has lower TK1 activity. Cytosolic TK1, also referred to as cellular TK1, is TK1 present inside cells and that can be isolated from such cells.

Human STK1, in clear contrast, can be in the form of high molecular weight complexes, such as oligomers or comprising such oligomers, having TK1 activity and dimer and tetramer forms having very low or even lacking TK1 activity. The oligomerization seems to be related to the formation of disulfide cross linking occurring in the blood.

TK1 material as used herein refers to TK1 in its various forms, such as dimers, tetramers, oligomers and complexes comprising TK1. The TK1 material is preferably a serum TK1 material, i.e., a STK1 material present in blood, blood plasma or serum in patient. The STK1 material may then comprise STK1 in the above mentioned forms, such as dimers, tetramers, oligomers and complexes comprising STK1. TK1 material also includes complexes with at least one TK1 protein unit and other molecules and/or macromolecules.

If at least one additional biomarker is determined, in addition to the TK1 material, the level(s) of at least one additional biomarker can be determined in the body sample, or a same type of body sample, as the TK1 material. In another embodiment, the at least one additional biomarker is determined in another body sample from the subject. For instance, serum TK1 material can be determined in a serum sample from the subject, whereas the at least one additional biomarker, such as HNL, can be determined in a plasma sample from the subject.

The level(s) of at least one additional biomarker can be determined according techniques well known in the art, including immunoassays using antibodies binding specifically to the respective biomarker. Illustrative, but non-limiting, examples of such immunoassays include ELISA or Particle-Enhanced Turbidimetric Immunoassay (PETIA)

In an embodiment, determining the level of TK1 material comprises contacting the body sample with the anti-TK1 antibody or the fragment thereof. This embodiment also comprises detecting or determining an amount of anti-TK1 antibody or fragment bound to the TK1 material.

Contacting the body sample with the antibody or the fragment thereof may be achieved by adding the antibody or the fragment thereof to the body sample and incubating the body sample with the antibody or the fragment thereof. The antibody or the fragment thereof thereby binds to the TK1 material forming a complex between the antibody or the fragment thereof and the TK1 material. In such an embodiment, measuring the amount of antibody or fragment bound to the TK1 material can be include measuring or quantifying the complex between the antibody or the fragment thereof and the TK1 material to thereby measure or quantify the amount of antibody or fragment bound to the TK1 material.

In an embodiment, determining the level of TK1 material comprises determining the level of TK1 material in the body sample based on the determined amount of bound anti-TK1 antibody or fragment and a standard correlation between an amount of bound anti-TK1 antibody or fragment and a level of TK1 material. Thus, in this embodiment a standard correlation is established between the amount of bound anti-TK1 antibody or fragment and a predefined concentration or level of TK1 material, such as a predefined concentration or level of recombinant TK1. Such a standard correlation can then be established by determining the respective amounts of bound anti-TK1 antibody or fragment for different predefined concentrations of the recombinant TK1 to get a correlation therebetween.

In an embodiment, the method comprises correlating the measured amount of antibody or fragment bound to the TK1 material to a level of TK1 material. This may be performed by using a pre-defined or standard correlation between measured amount of antibody or fragment bound to a reference TK1 material and concentration of the reference TK1 material. A typical reference TK1 material that can be used when generating such a pre-defined or standard correlation is recombinant human TK1.

The pre-defined or standard correlation may, thus, be generated by adding the antibody or the fragment thereof to different samples comprising different concentrations of the reference TK1 material, preferably recombinant human TK1. The amount of antibody or fragment bound to the reference TK1 material, preferably recombinant human TK1, is then measured in the different samples to thereby get a standard curve, function or relationship between concentration of reference TK1 material, preferably recombinant human TK1, and the measured amount of antibody or fragment bound to the reference TK1 material, preferably recombinant human TK1.

This pre-defined or standard correlation, such as standard curve, function or relationship, can then be used to map or convert the measured amount of antibody or fragment bound to the TK1 material in the body sample to a concentration of the TK1 material in the body sample.

In an embodiment, the body sample is processed prior to or during the incubation of the body sample with the antibody or the fragment thereof. This sample processing may be used to stabilize selected TK1 forms in the body sample and/or to break larger TK1 complexes or oligomers into smaller complexes or multimers.

Hence, in an embodiment, a sample dilution or pretreatment buffer is added to the body sample prior to or in connection with adding the antibody or the fragment thereof to the body sample, preferably prior to adding the antibody or the fragment thereof to the body sample.

In an embodiment, the sample dilution buffer comprises ATP, preferably in a concentration selected within an interval of from 1.5 mM up to 50 mM. As previously described herein, ATP stabilizes the tetramer form of TK1, which has high enzymatic TK1 activity.

In another embodiment, the sample dilution buffer comprises a reducing agent. The reducing agent may then break disulfide cross links in larger TK1 complexes and oligomers to obtain smaller TK1 forms, such as tetramers. Various reducing agents capable of breaking disulfide bonds can be used according to the embodiments including, but not limited to, dithioerythritol (DTE), dithiothreitol (DTT), dithiobutylamin (DTBA), tris(2-carboxyethyl)phosphine) (TCEP), and combinations thereof. The amount of the reducing agent is typically selected within an interval of from 0.1 mM up to 10 mM.

The sample dilution buffer may, in an embodiment, comprise both ATP and a reducing agent.

In an embodiment, the anti-TK1 antibody is a monoclonal anti-TK1 antibody. In another embodiment, the anti-TK1 antibody is a polyclonal anti-TK1 antibody.

In an embodiment, the anti-TK1 antibody has specificity for an epitope or peptide consisting of an amino acid sequence from the C-terminal region of TK1, preferably of human TK1.

The peptide is preferably selected from a portion of TK1 ranging from amino acid position 200 to the end of the TK1, i.e., amino acid position 234 in humans (SEQ ID NO: 28). In a particular embodiment, the peptide is selected from a portion of the TK1 protein ranging from amino acid position 205, preferably 210 to amino acid position 230, preferably 225.

The peptide is preferably an N-mer, wherein N is an integer within a range of from 8 up to 20, preferably within a range of from 10 up to 15.

The peptide preferably consists of N consecutive amino acids in the C-terminal region of the TK1 protein.

In an embodiment, the peptide consists of the following amino acid sequence GEAVAARKLF (SEQ ID NO: 1). In another embodiment, the peptide consists of the following amino acid sequence NCPVPGKPGE (SEQ ID NO: 2). In a further embodiment, the peptide consists of the following amino acid sequence PVPGKPGEAV (SEQ ID NO: 3). In yet another embodiment, the peptide consists of the following amino acid sequence NCPVPGKPGEAV (SEQ ID NO: 4).

A monoclonal anti-TK1 antibody having specificity for an epitope consisting of GEAVAARKLF (SEQ ID NO: 1) has a variable heavy (VH) domain complementarity determining region 1 (CDR1) having amino acid sequence DYEMH (SEQ ID NO: 5), a VH domain CDR2 having amino acid sequence AIHPGYGGTAYNQKFKG (SEQ ID NO: 6), a VH domain CDR3 having amino acid sequence FITKFDY (SEQ ID NO: 7), a variable light (VL) domain CDR1 having amino acid sequence KSSQSLLDSDGKTFLN (SEQ ID NO: 8), a VL domain CDR2 having amino acid sequence LVSKLDS (SEQ ID NO: 9) and a VL domain CDR3 having amino acid sequence WQGTHFPWT (SEQ ID NO: 10).

A monoclonal anti-TK1 antibody having specificity for the epitopes NCPVPGKPGE (SEQ ID NO: 2), PVPGKPGEAV (SEQ ID NO: 3) and NCPVPGKPGEAV (SEQ ID NO: 4) has a VH domain CDR1 having amino acid sequence DYEMH (SEQ ID NO: 5), a VH domain CDR2 having amino acid sequence AILPGSGGTAYNQKFKG (SEQ ID NO: 17), a VH domain CDR3 having amino acid sequence LITTFDY (SEQ ID NO: 18), a VL domain CDR1 having amino acid sequence KSSQSLLDSDGKTYLN (SEQ ID NO: 19), a VL domain CDR2 having amino acid sequence LVSKLDS (SEQ ID NO: 9), and a VL domain CDR3 having amino acid sequence WQGTHFPWT (SEQ ID NO: 10).

In another embodiment, the anti-TK1 antibody has specificity for a conformation dependent epitope of human TK1. A monoclonal anti-TK1 antibody having specificity for such a conformation dependent epitope has a VH domain CDR1 having amino acid sequence SGYSWH (SEQ ID NO: 11), a VH domain CDR2 having amino acid sequence YIHYSGSTTYNPSLKG (SEQ ID NO: 12), a VH domain CDR3 having amino acid sequence WGTGHWYFDV (SEQ ID NO: 13), a VL domain CDR1 having amino acid sequence RSSTGAVTTTNYAN (SEQ ID NO: 14), a VL domain CDR2 having amino acid sequence GTNNRVP (SEQ ID NO: 15), and a VL domain CDR3 having amino acid sequence ALWYSNHWV (SEQ ID NO: 16).

The above-three presented examples of monoclonal anti-TK1 antibodies that can be used according to the embodiments are further disclosed in WO 2015/094106.

Hence, in an embodiment, the monoclonal antibody or the fragment thereof is selected from the group consisting of a monoclonal antibody or a fragment thereof having specificity for GEAVAARKLF (SEQ ID NO: 1) of human TK1, a monoclonal antibody or a fragment thereof having specificity for at least one of NCPVPGKPGE (SEQ ID NO: 2), PVPGKPGEAV (SEQ ID NO: 3) and NCPVPGKPGEAV (SEQ ID NO: 4) of human TK1, and a monoclonal antibody or a fragment thereof having specificity for a conformation dependent epitope of human TK1.

In another embodiment, the anti-TK1 antibody has specificity for an epitope or peptide consisting of KPGEAVAARKLFAPQ (SEQ ID NO: 20).

An anti-TK1 antibody having specificity for this epitope is further disclosed in WO 95/29192.

At least one additional amino acid, such as a cysteine residue, may be added to the N-terminal or C-terminal, preferably the N-terminal, of the peptide for use as coupling to other molecules, such as carrier proteins.

In a further embodiment, the anti-TK1 antibody has specificity for an epitope or peptide consisting of an amino acid sequence from an active site of TK1. The peptide is preferably selected from a portion of TK1 ranging from amino acid position 150 to amino acid position 190 in human TK1. In a particular embodiment, the peptide is selected from a portion of TK1 ranging from amino acid position 155, preferably 160 and more preferably 161, to amino acid position 185, preferably 183.

The peptide is preferably an M-mer, wherein M is an integer within a range of from 10 up to 40, preferably within a range from 20 up to 30 and more preferably 23 or 24.

The peptide preferably consists of M consecutive amino acids in the active site of the TK1 protein.

At least one additional amino acid, such as a cysteine residue, may be added to the N-terminal or C-terminal, preferably the N-terminal, of the peptide for use as coupling to other molecules, such as carrier proteins.

In an embodiment, the peptide consisting of an amino acid sequence from the active site of TK1 has an amino acid sequence corresponding to amino acid positions 161 to 183 in human TK1, i.e., has amino acid sequence of AYTKRLGTEKEVEVIGGADKYHS (SEQ ID NO: 21).

An anti-TK1 antibody having specificity for this epitope is further disclosed in WO 2008/142664.

In a further embodiment, the antibody or the fragment thereof is a monoclonal antibody or a fragment thereof as disclosed in WO 2019/201901.

For instance, the monoclonal antibody could be mAb 6C6, mAb 4H4 or mAb 23C11.
mAb 6C6 VH domain (SEQ ID NO: 22): 
METGLRWLLLVAVLKGVQCQEQLEESGGDLVKPEGSLTLTCTASRFSFSSSYWICWVRQAPGKGLEW
IACIYAGDSGSSYYASWAKGRFTVSKTSSTTVTLQTTSLTAADTATYFCARASVGAAYDYFALWGPGTL
VTVSSGQPKAPSVFPLAPCCGDTPSSTVTLGCLVKGYLPEPVTVTWNSG
mAb 6C6 VL domain (SEQ ID NO: 23): 
MDTRAPTQLLGLLLLWLPGARCALVMTQTPASVEAAMGGTVTIKCQASEDVSSHLAWYQQRPGQPPK
LLIYGASDLASGVPSRFTGSGSGTQFTLAISDLECADAATYYCQGYYYISDSPYVFGGGTEVVVKGDPV
APTVLIFPPAADQVATGTVTIVCVANKYFPDVTVTWEVDGTTQTTGIENSKTPQNSADCTYNLSSTLTLT
STQYNSHKEYTCKVTQGTTSVVQSFNRGDC
mAb 4H4 VH domain (SEQ ID NO: 24): 
METGLRWLLLVAVLKGVQCQSLEESGGGLVQPEGSLTLTCTASGFSFSSGYDMCWVRQTPGKGLEW
IACISVDSDGVTYYASWAKGRFTISKTSSTTVTLQMTSLTAADTATYFCARGYESSSGVYIPYFTLWGP
GTLVTVSSGQPKAPSVFPLAPCCGDTPSSTVTLGCLVKGYLPEPVTVTWNSG
mAb 4H4 VL domain (SEQ ID NO: 25): 
MDMRAPTQLLGLLLLWLPGARCADIVLTQTPASVEAAVGGTVTIKCQASQSIYSYLAWYQHKPGQPPK
LLIYKASTLASGVPSRFKGSGSGTEYTLTISDLECADAATYYCQHYYYSSTSGGGVFGGGTEVVVKGD
 
PVAPTVLIFPPAADQVATGTVTIVCVANKYFPDVTVTWEVDGTTQTTGIENSKTPQNSADCTYNLSSTLT
LTSTQYNSHKEYTCKVTQGTTSVVQSFNRGDC
mAb 23C11 VH domain (SEQ ID NO: 26): 
METGLRWLLLVAVLKGVQCQSLEESGGRLVTPGTPLTLTCTASGFSLSNYYMSWVRQAPGKGLEWIGI
IYGDDNTYCANWTKGRFTISKTSTTVDLTITSPTTEDTATYFCARGPDYIAAKMDIWGPGTLVTVSLGQP
KAPSVFPLAPCCGDTPSSTVTLGCLVKGYLPEPVTVTWNSG
mAv 23C11 VL domain (SEQ ID NO: 27):

MDTRAPTQLLGLLLLWLPGARCDVVMTQTPASVEAAVGGTVTIKCQASQSISGYLSWYQQKPGQRPK
LLIYRASTLESGVPSRFKGSGSGTEFTLTISDLECADAATYYCQCTYGSSTFSSYGNAFGGGTEVVVKG
DPVAPTVLIFPPAADQVATGTVTIVCVANKYFPDVTVTWEVDGTTQTTGIENSKTPQNSADCTYNLSST
LTLTSTQYNSHKEYTCKVTQGTTSVVQSFNRGDC

In an embodiment, the level of TK1 material in the body sample is determined using a kit. The kit preferably comprises a first, or so-called capture, anti-TK1 antibody immobilized to a support or intended to be immobilized to the support and a second, or so-called detection, anti-TK1 antibody. The first and second anti-TK1 antibodies can be selected from the above described illustrative examples of monoclonal and polyclonal anti-TK1 antibodies.

In a particular embodiment, the kit comprises a first monoclonal antibody or a first fragment thereof having specificity for an epitope selected from the group consisting of i) GEAVAARKLF (SEQ ID NO: 1) of human TK1, ii) at least one of NCPVPGKPGE (SEQ ID NO: 2), PVPGKPGEAV (SEQ ID NO: 3) and NCPVPGKPGEAV (SEQ ID NO: 4) of human TK1, and iii) a conformation dependent epitope of human TK1. The kit also comprises a second monoclonal antibody or a second fragment thereof having specificity for an epitope selected from the group consisting of i) GEAVAARKLF (SEQ ID NO: 1) of human TK1, ii) at least one of NCPVPGKPGE (SEQ ID NO: 2), PVPGKPGEAV (SEQ ID NO: 3) and NCPVPGKPGEAV (SEQ ID NO: 4) of human TK1, and iii) a conformation dependent epitope of human TK1.

In an embodiment, the first antibody or the first fragment thereof is a so-called capture antibody immobilized to a support or intended to be immobilized to the support and the second antibody or the second fragment thereof is a so-called detection antibody. In another embodiment, the second antibody or the second fragment thereof is the capture antibody immobilized to the support or intended to be immobilized to the support, whereas the first antibody or the first fragment thereof is used as detection antibody.

In an embodiment, the first and second anti-TK1 antibodies have specificities for different epitopes in human TK1.

In another embodiment, the first and second anti-TK1 antibodies have specificities for the same epitope in human TK1. This is possible since the same epitope may be present at multiple copies in the high molecular weight complexes of multiple TK1 protein units. Thus, the TK1 material may be a multivalent complex of multiple, i.e., at least two, TK1 protein units.

Hence, TK1 material as used herein includes TK1 protein, complexes with multiple TK1 protein units, such as dimers, trimers, tetramers, etc. TK1 material also includes complexes with at least one TK1 protein unit and other molecules and/or macromolecules.

In an embodiment, one of the first and the second anti-TK1 antibody has specificity for a peptide consisting of an amino acid sequence from the active site of TK1 and the other of the first and second anti-TK1 antibody has specificity for a peptide consisting of an amino acid sequence from the C-terminal region of TK1.

In another embodiment, one of the first and the second anti-TK1 antibody has specificity for a peptide consisting of a first amino acid sequence from the C-terminal region of TK1 and the other of the first and the second anti-TK1 antibody has specificity for a peptide consisting of the first amino acid sequence from the C-terminal region of TK1 or a second, different amino acid sequence from the C-terminal region of TK1.

In a further embodiment, one of the first and the second anti-TK1 antibody has specificity for a peptide consisting of an amino acid sequence from the C-terminal region of TK1 and the other of the first and the second anti-TK1 antibody has specificity for a conformation dependent epitope of human TK1.

A fragment of an antibody as used herein can be selected from a group consisting of a single chain antibody, a Fv fragment, a scFv fragment, a Fab fragment, a F(ab')2 fragment, a Fab' fragment, a Fd fragment, a single-domain antibody (sdAb), a scFv-Fc fragment, a di-scFv fragment and a CDR region.

The antibody or the fragment thereof specifically binds to the TK1 material, and in particular binds specifically to the TK1 protein.

The specificity of an antibody can be determined based on affinity and/or avidity. The affinity, represented by the equilibrium constant for the dissociation of an antigen with the antibody (*K*_{D}), is a measure for the binding strength between an antigenic determinant and an antigen-binding site on the antibody. The lesser the value of *K*_{D}, the stronger the binding strength between the antigenic determinant and the antibody. Alternatively, the affinity can also be expressed as the affinity constant (*K*_{A}), which is 1/*K*_{D}. As will be clear to the skilled person, affinity can be determined in a manner known per se, depending on the specific antigen of interest.

Avidity is the measure of the strength of binding between an antibody and the pertinent antigen. Avidity is related to both the affinity between an antigenic determinant and its antigen binding site on the antibody and the number of pertinent binding sites present on the antibody.

Typically, antibodies will bind to their antigen with a dissociation constant (*K*_{D}) of 10⁻⁵ to 10⁻¹² moles/liter (M) or less, and preferably 10⁻⁷ to 10⁻¹² M or less and more preferably 10⁻⁸ to 10⁻¹² M, i.e. with an association constant (*K*_{A}) of 10⁵ to 10¹² M⁻¹ or more, and preferably 10⁷ to 10¹² M⁻¹ or more and more preferably 10⁸ to 10¹² M⁻¹.

Generally, any *K*_{D} value greater than 10⁻⁴ M (or any *K*_{A}, value lower than 10⁴ M⁻¹) is considered to indicate non-specific binding.

Preferably, an anti-TK1 antibody or fragment of the embodiments will bind to the serum form of human TK1 with an affinity less than 500 nM, preferably less than 200 nM, more preferably less than 10 nM, such as less than 5 nM.

Specific binding of an antibody or a fragment thereof to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art.

In an embodiment, the kit is a sandwich assay kit. In a particular embodiment, the kit is ELISA kit and preferably a sandwich ELISA.

In the discussion below, the first anti-TK1 antibody or first fragment is assumed to be the capture antibody with the second anti-Tk1 antibody or second fragment acting as detection antibody. The embodiments are, however, not limited thereto but could switch capture and detection antibodies.

A sandwich ELISA can be used to detect TK1 protein in a biological sample by preparing a surface of a support, such as a solid support, to which the first anti-TK1 antibody is bound as so-called capture antibody. In a preferred embodiment, a known quantity of the first anti-TK1 antibody is bound to the surface of the support. Any nonspecific binding sites on the surface are optionally but preferably blocked. The biological sample is then applied to the surface so that any TK1 protein present therein will be captured by the immobilized first anti-TK1 antibodies. Unbound material is preferably removed by one or multiple washing steps. The second anti-TK1 antibody, typically denoted detection antibody, is then added and is allowed to bind to any TK1 protein captured by the first anti-TK1 antibody.

The amount of bound second anti-TK1 antibody is then determined by direct or indirect detection methods. For instance, a label or enzyme can be attached directly to the second anti-TK1 antibody or indirectly via a link, such as a biotin-streptavidin or a biotin-avidin link. It is, alternatively, possible to use a secondary antibody that is labeled or connected to an enzyme and binds specifically to the second anti-TK1 antibody.

Hence, in an embodiment the second anti-TK1 antibody has a covalently attached biotin. Alternatively, the second anti-TK1 antibody has a covalently attached streptavidin or avidin.

The kit preferably also comprises a horseradish peroxidase (HRP) labeled streptavidin or a HRP labeled avidin. Alternatively, the kit also comprises a HRP labeled biotin. The kit also comprises a HRP substrate, such as a 3,3',5,5'-tetramethylbenzidine (TMB) substrate, a 3,3'-diaminobenzidine (DAB) substrate or a 2,2'-azino-bis(3-ethylbenzothiazoline-6-sulphonic acid) (ABTS) substrate. In such a case, the level of TK1 protein in the sample can be determined by spectrophotometric methods that detect the conversion of the chromogenic substrate by HRP into a colored product that is detectable.

In an embodiment, the kit also comprises a microtiter plate (MCP) as the support to which the first anti-TK1 antibody is immobilized or is intended to be immobilized.

A suitable ELISA kit that can be used according to the embodiments is the AroCell TK 210 ELISA produced by AroCell AB, Uppsala, Sweden.

The kit does not necessarily have to be an ELISA kit. In another embodiment, the kit uses affinity chromatography where the first anti-TK1 antibody is bound to the stationary phase, such as to a gel matrix or beads in a column. For instance, the gel matrix or beads could be made of agarose, such as SEPHAROSE^{®}.

In such a case, TK1 protein present in a biological sample will be entrapped in the column through binding to the immobilized first anti-TK1 antibodies. Following washing, the bound TK1 protein can be eluted and detected using the second anti-TK1 antibody. For instance, the amount of eluted TK1 protein can be determined using Western blotting and with the second anti-TK1 antibody for TK1 detection using direct or indirect detection methods.

The support could alternatively be magnetic beads, such as DYNABEADS^{®} magnetic beads.

The kit does not necessarily have to include two anti-TK1 antibodies but could instead include only one type of anti-TK1 antibodies.

Furthermore, the kit does not necessarily have to comprise a so-called capture anti-TK1 antibody. In clear contrast, multiple, i.e., at least two, different anti-TK1 antibodies could be used to determine the level of TK1 material without the need for immobilizing at least one of the anti-TK1 antibodies.

In an embodiment, the patient is a human patient and preferably an adult human patient.

In an embodiment, the method also comprises selecting a treatment for the patient based on the estimated likelihood, the diagnosis or the classification obtained based on the determined level of TK1 material in the body sample from the patient.

Thus, an optimal or at least suitable treatment is selected for the patient based on the determined level of TK1 material in the body sample and thereby based on whether the respiratory infection of the patient is predicted to be caused by *M. pneumoniae,* i.e., whether the patient is likely to suffer from *Mycoplasma* pneumonia, or by a virus or other bacteria, i.e., whether the patient is instead likely to suffer from a viral pneumonia or a bacterial pneumonia caused by a bacterium other than *M*. *pneumoniae.* This means that patients with a high determined level of TK1 material and thereby predicted to suffer from *Mycoplasma* pneumonia could be selected for a treatment adapted for combating *M. pneumoniae* infections, whereas patients with a comparatively lover level of TK1 material could instead be selected for a treatment adapted for combating viral or other bacterial infections. Examples of treatments adapted for *Mycoplasma* pneumonia include antibiotics targeting the bacterial rRNA in ribosomal complexes, including macrolides, tetracycline, ketolides, and fluoroquinolone. The difficulty in eradicating *Mycoplasma pneumoniae* infections is due to the ability of the bacterium to persist within an individual, as well as the lack of cell wall in *M. pneumoniae,* which renders multiple antibiotics directed at the bacterial cell wall ineffective in treating infections. *M. pneumoniae* therefore displays resistance to antimicrobials, such as β-lactams, glycopeptides, sulfonamides, trimethoprim, polymixins, nalidixic acid, and rifampin.

As previously mentioned herein, TK1 protein determination according to the embodiments can be combined with at least one other biomarker for *M*. *pneumoniae* including, but not limited to, CRP, PCT, calprotectin and B-HNL and/or P-HNL. The method may then comprise at least one additional step of determining a level of CRP, PCT, calprotectin and/or B-HNL and/or P-HNL in a body sample obtained from the subject. In such an embodiment, estimating the likelihood comprises estimating a likelihood that the respiratory infection is *Mycoplasma* pneumonia caused by *Mycoplasma pneumoniae* based on the determined level of TK1 material and the determined level of CRP, PCT, calprotectin and/or B-HNL and/or P-HNL. Correspondingly, diagnosing the subject comprises, in such an embodiment, diagnosing the subject with *Mycoplasma* pneumonia based on the determined level of TK1 material and the determined level of CRP, PCT, calprotectin and/or B-HNL and/or P-HNL.

### EXAMPLES

### EXAMPLE 1

The sandwich AroCell TK 210 ELISA produced by AroCell AB, Uppsala, Sweden was used as an alternative to TK1 activity assay. This ELISA is a quantitative enzyme immunoassay based on the sequential addition of a sample, a biotin labeled anti-TK1 monoclonal antibody, a streptavidin-labeled enzyme-conjugate and a substrate to microtiter wells coated with another monoclonal anti-TK1 antibody [4].

The purpose of the present study was to investigate if acute infections and more specifically lower respiratory tract infections with *M. pneumoniae* gave altered serum TK1 protein concentrations compared to those caused by other viral and bacterial infections. The inclusion criteria for patients in the study were fever of >38°C and signs and symptoms of an acute infection. One control group was 144 apparently non-infected healthy subjects and a second control group was 98 patients with clinical diagnosis of bacterial infection. Lower respiratory tract infection was verified by X-ray examination of the lungs as well as CRP, white blood cell counts, and in some cases, microbiological test results. In the pneumonia group, the diagnosis was verified with a positive chest X-ray and supported by a positive polymerase chain reaction (PCR) test for *M*. *pneumoniae* from the lower respiratory tract samples [5].

The results clearly showed significantly increased levels of serum TK1 protein in patients with acute *M*. *pneumoniae* infection both in comparison with healthy subjects as well as subjects with viral acute infections as well as lower respiratory infection caused by bacteria. Thus, these results indicated that TK1 protein determinations served as a valuable biomarker for the difficult to diagnose *M*. *pneumoniae* infection, complementing or replacing other biomarkers, such as CRP and B-HNL.

### Materials and Methods

### Patients and diagnosis of different forms of infections

One hundred sixty-two patients presented with symptoms of acute infection were judged on clinical grounds to have either a bacterial (n=98) or viral (n=64) cause of their infection, and they were given a clinical diagnosis. The 98 patients with clinical diagnosis of bacterial infection comprised of 55 women (median age 37 years; range 18 to 84 years) and 43 men (median age 48 years; range 20 to 90 years).

The inclusion criteria were fever of at least 38°C and signs and symptoms of acute respiratory infection. Known to the adjudicator were, in addition to clinical findings, CRP, white blood cell counts, X-ray findings, and in some cases, microbiological test results. Based on this information, patients were judged to have either a bacterium or virus as their infectious agent, i.e., they were given a clinical diagnosis of infection. Lower respiratory tract infection was verified by X-ray examination of the lungs. Exclusion criterion was known chronic viral infection, such as human immunodeficiency virus (HIV) infection.

### AroCell TK 210 ELISA procedure

The TK1 protein levels in serum samples were determined using the AroCell TK 210 ELISA according the manufacturer's instructions [4, 6]. In brief, serum samples, calibrators and controls were pre-incubated with a sample dilution buffer (SDB) for 1 h at room temperature. Antibody pre-coated plates were washed with wash buffer before addition of incubated serum samples, calibrators and controls. After 2 hours of incubation on a shaking platform, the plates were washed 4 times with wash buffer. Then the plates were incubated with biotinylated anti-TK1 antibody for 1 h on a shaking platform. After another cycle of washing, plates were incubated with enzyme-labeled streptavidin for 30 min. After the final wash, a substrate solution was added and the color intensity was measured by a spectrophotometer at 450 nM. Serum TK1 protein levels in serum samples were calculated using the calibrators as a standard and the SDB as blank with the 4-PL curve fit program. Each sample was analyzed in duplicates and the mean value was expressed as µg/L.

### Statistical methods

Data were expressed as medians and interquartile ranges. Comparisons of groups were performed by the nonparametric Mann-Whitney test for independent groups. In order to estimate the clinical performances of the biomarker assays, receiver operating characteristic (ROC) analyses were performed, and comparisons of the areas under curve (AUC) were analyzed by c-statistics.

For calculations of the statistics, MedCalc Statistical Software version 16.4.3 (MedCalc Software bvba, Ostend, Belgium [https://www.medcalc.org]; 2016) was used.

### Results

Fig. 1 shows the TK1 protein concentrations in healthy subjects, and in patients with bacterial, viral or *Mycoplasma pneumoniae* infections. In a pairwise comparison between healthy and the patient groups significant differences with P values of <0.0001 were observed. When the patients groups were limited to patients with upper respiratory tract infections caused by bacteria or *M*. *pneumoniae* the difference between the groups were still highly significant both against healthy and pneumonia caused by bacteria, see Fig. 2.

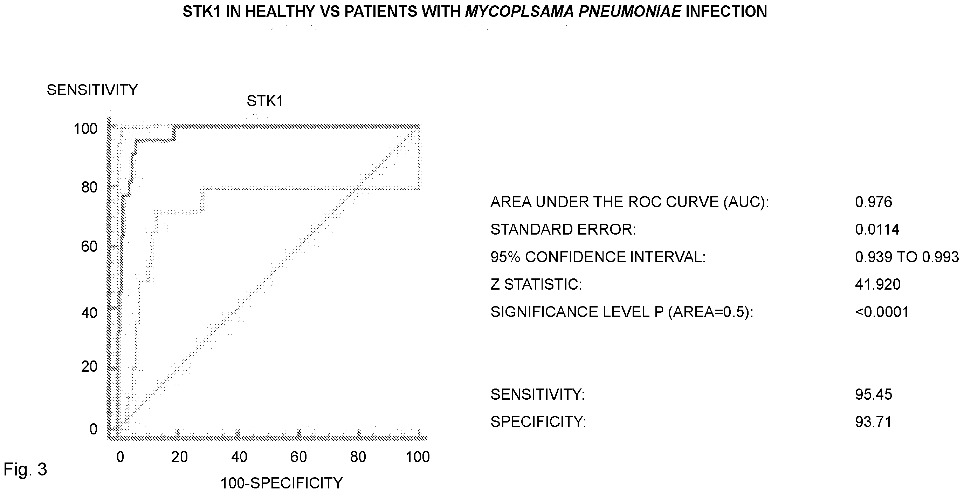

A ROC curve analysis was performed to estimate the potential clinical performance of the TK1 biomarker in differentiating the *M*. *pneumoniae* patient group from the healthy controls, see Fig. 3. The area under curve (AUC) value was 0.98 and the sensitivity and specificity were 95 % and 94 %, respectively.

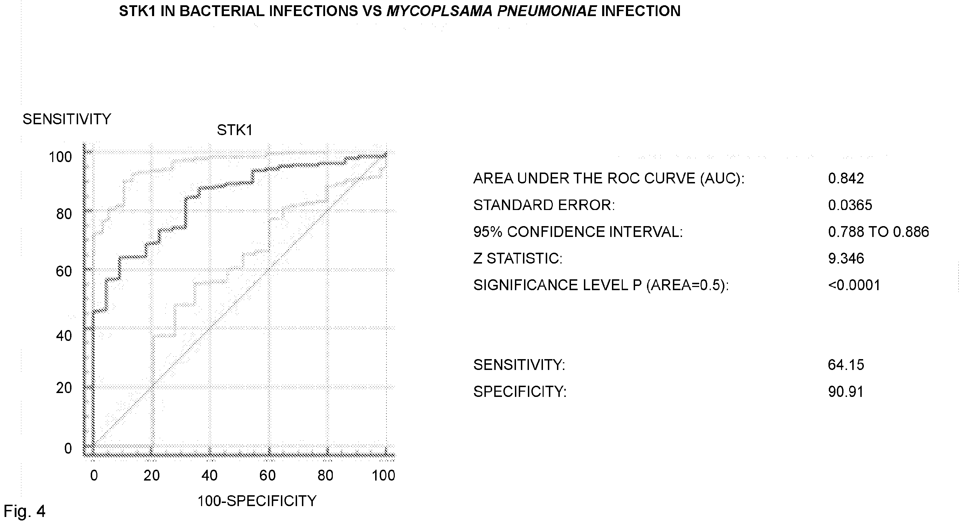

When the *M. pneumoniae* group instead was compared to patients with acute bacterial infections the AUC value was 0.84 and the sensitivity and specificity were 64 % and 90 %, respectively, see Fig. 4.

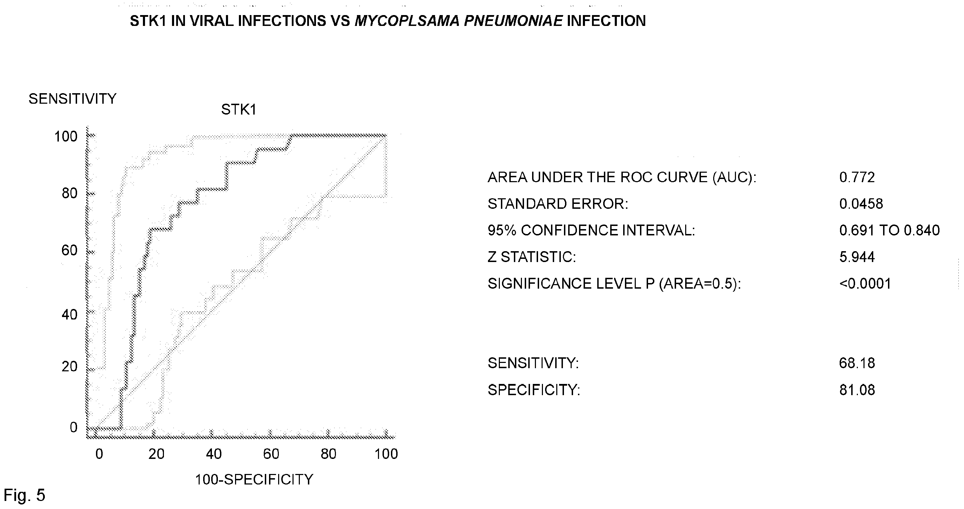

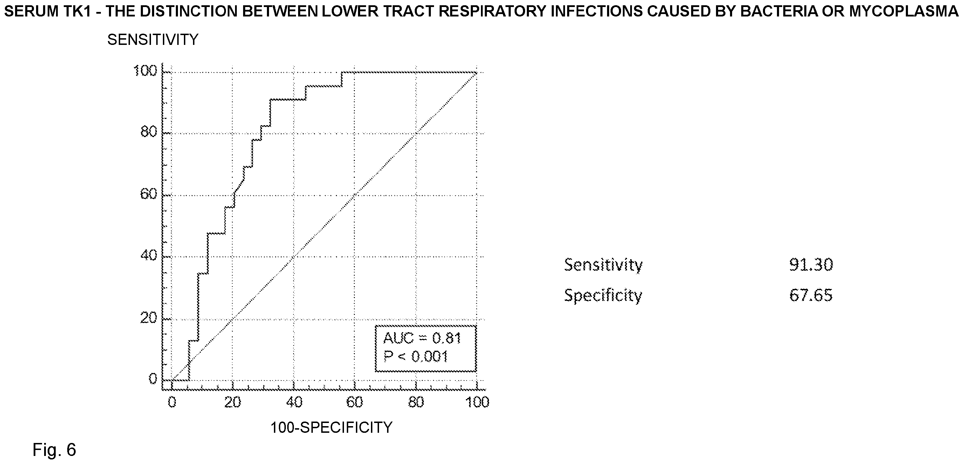

Fig. 5 shows the ROC curve analysis for the *M. pneumoniae* group compared to the patients with acute infection caused by viral agents. In this experiment the AUC value was 0.77 and the sensitivity was 68 % and specificity was 81 %. When instead the *M. pneumoniae* group was compared to patients with bacterial lower respiratory tract infections, the AUC value was 0.81 and the sensitivity and specificity were 91 % and 68 %, respectively, see Fig. 6.

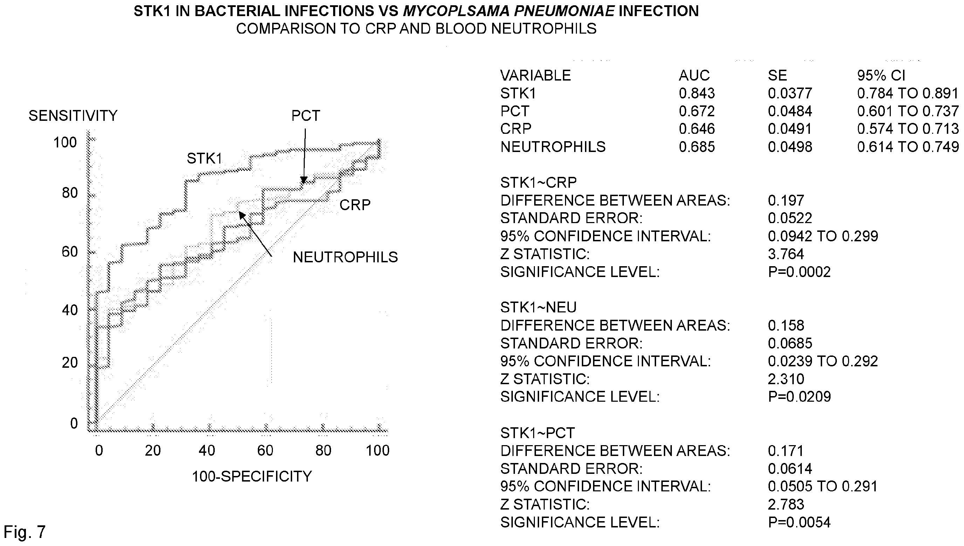

CRP and procalcitonin (PCT) as well as the neutrophil blood counts have been used as biomarkers in patients with bacterial infections. In Fig. 7 a ROC curve analysis is presented where these biomarkers are compared with TK1. It is clear from these results that TK1 is superior to any of the other biomarkers with regard to differentiating between bacterial and *M. pneumoniae* infections with differences that are significant with P values from <0.02 to <0.0002.

The mechanisms leading to up-regulation of serum TK1 in *M*. *pneumoniae* infections are not known. *M*. *pneumoniae* is a wall-less bacteria and thereby lacks lipopolysaccharide antigens. Hence, the mechanism is different than for most other bacteria. It has been demonstrated that *M*. *pneumoniae* induces efflux of ATP from host cells and this can activate inflammasomes via the P2X7 receptor, which is followed by the secretion of IL-1β. Other studies also demonstrated that cytoadherence of *M*. *pneumoniae* to the host cells is strongly associated with the induction of inflammatory responses. Thus, there is evidence that *M*. *pneumoniae* induce host response mechanisms different from those of other bacteria.

The results presented in Figs. 1 to 7 strongly indicate that serum TK1 protein determinations may serve as a sensitive blood biomarker for distinguishing *M. pneumoniae* infections from other cause of infections, such as viral or bacterial agents. TK1 as biomarker was clearly superior to CRP, PCT and neutrophil counts. Therefore, the inclusion of TK1 concentration as a biomarker, particularly in case of lower tract respiratory disease, could significantly improve the *in vitro* diagnostics of a large group of patients seeking primary medical care.

### EXAMPLE 2

The correct diagnosis of acute infections as to bacteria, mycoplasma or virus is a clinical challenge and has a great impact on the therapeutic decisions. Current diagnostic tests of *Mycoplasma pneumoniae* infections of the respiratory tract, such as PCR and serology, are either somewhat unreliable or slow and do not entirely meet the clinical needs of accurate and fast diagnosis. The aim of this Example was to examine a panel of candidate biomarkers and their capacity to distinguish *M. pneumoniae* respiratory infections from respiratory infections caused by either bacterial or virus.

### Materials and Methods

The study group comprised 144 healthy controls with an average age of 43.6 ±12.8 years consisting of 57 males (age 41.3±12.7 years) and 87 females (age 45.0 ±12.8 years) and 156 patients with confirmed etiology of their acute respiratory infection. The inclusion criteria were fever >38°C and signs and symptoms of acute respiratory infections. The clinical diagnosis of the causes of the infection was complemented with objective microbiological/serological testing. Blood was drawn before start of antibiotics treatment. The study was approved by the ethics committee of Uppsala.

Of these 156 patients 86 had a bacterial infection, men n=41 (age 54 years IQ range 41-71) and women n=45 (age 43 years IQ range 29-66); 42 had a viral infection, men n=23 (age 49 IQ range 39-73) and women n=19 (age 40 IQ range 25-57); and 28 had *M. pneumoniae* infection, men n=14 (age 42.5 years IQ range 34-61) and women n=14 (age 36.5 years IQ range 22-44). The estimated duration of the mycoplasma infection was 168 hours, 130-196 hours (median and IQ range).

### Biomarker assays

HNL was measured in EDTA-plasma (Diagnostics Development, Uppsala, Sweden) by an HNL assay configured to specifically catch the dimer of the molecule and subsequently called P-HNL Dimer. Calprotectin (Gentian AS, Norway) was measured in EDTA-plasma. PCT (Thermo Fisher Scientific Frederick, MD, USA) and TK1 (AroCell AB, Uppsala, Sweden) were measured in serum. All biomarkers were run according to the manufacturer's instructions for use. Imprecision of duplicate samples were between 4-10% CV for all assays.

### Statistics

Data was expressed as medians and interquartile ranges or full ranges as indicated. Comparisons of groups were performed by the non-parametric Mann-Whitney's test for independent groups or the Kruskal-Wallis ANOVA. The clinical performances of the biomarker assays were estimated by receiver operating characteristics (ROC) analyses. The diagnostic performance of combinations of biomarkers was expressed as Area Under the ROC-curve (AuROC) and calculated by logistic regression analysis. For the calculations of the statistics, MedCalc Statistical Software version 19.1.5 (MedCalc Software bvba, Ostend, Belgium; http://www.medcalc.org; 2020) was used.

### Results

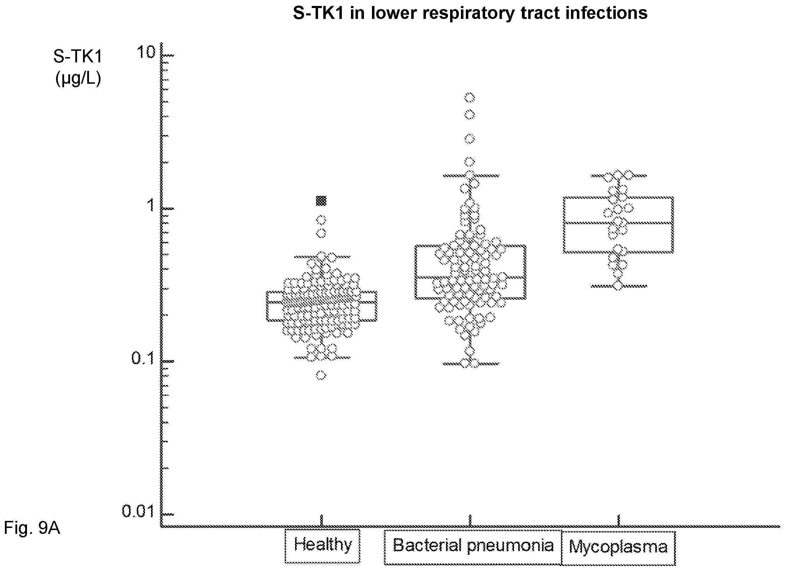

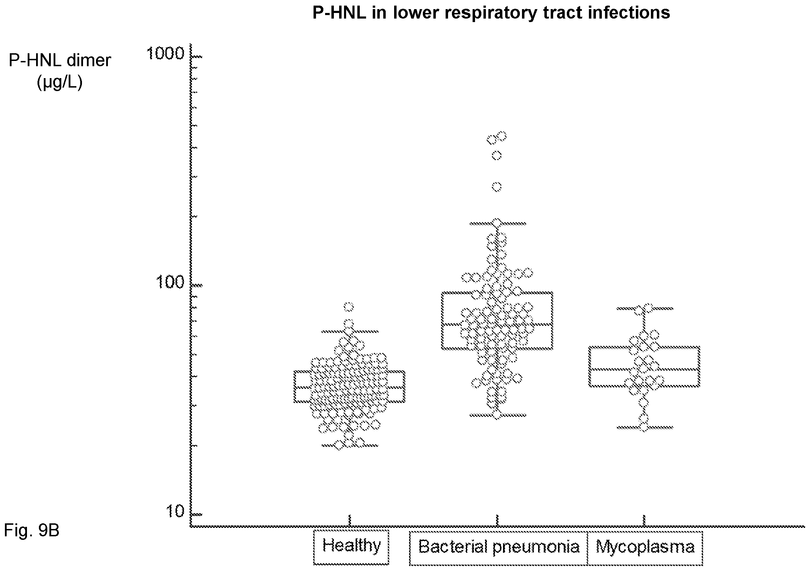

Figs. 9A and 9B show the distributions in plasma/serum of the TK1 and HNL dimer in patients with acute respiratory infections caused by *Mycoplasma pneumoniae* or bacteria. As compared to the findings in healthy subjects, S-TK1 was significantly elevated in patients with *M. pneumoniae* infections. As compared to bacterial infections, the concentrations of S-TK1 (p<0.001) was elevated in *M. pneumoniae* infections, whereas the concentrations of P-HNL Dimer (p<0.001) was significantly lower.

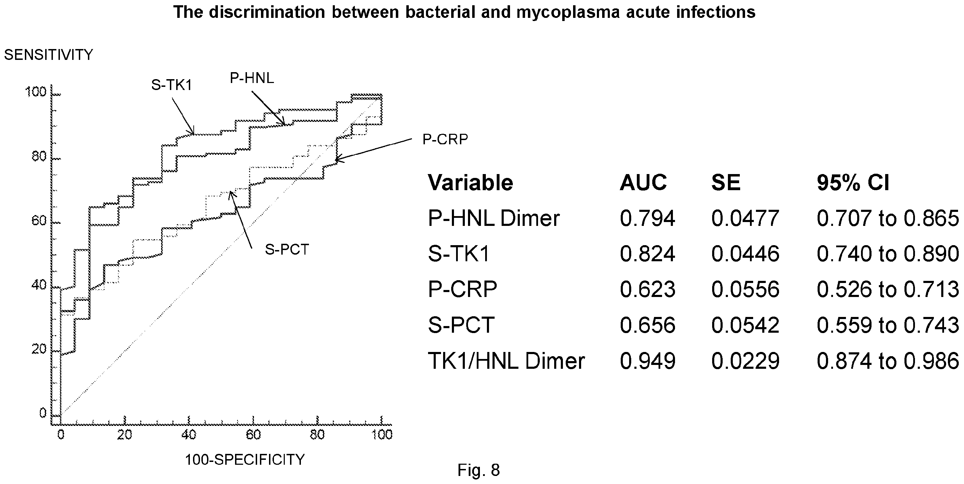

The distinction between *M. pneumoniae* respiratory infections from respiratory infections caused by bacteria was estimated by ROC curve analysis. As shown in Fig. 8 all biomarkers showed a significant distinction. P-HNL Dimer and S-TK1 stood out with AUCs of 0.82 and 0.79, respectively.

Based on the results from the above ratios were constructed between selected biomarkers. For the discrimination between *M. pneumoniae* and bacterial respiratory infections, the ratio between the results with of TK1 and HNL dimer in serum and plasma, respectively, increased the AUC over either of these biomarkers to 0.95.

The clinical diagnosis of *Mycoplasma* pneumonia from pneumonia caused by bacteria is a challenge and relies currently on clinical signs and symptoms, PCR-based diagnosis and serology. Mild *Mycoplasma* pneumonia may progress into severe disease why early detection and treatment is important. In this Example, the results of selected different blood-based biomarkers are presented together with their capacity to distinguish *Mycoplasma* pneumonia from lower respiratory tract infections caused by bacteria. In the distinction to bacterial infections, TK1 and the dimeric form of HNL seemed of particular interest. The concentration of TK1 was increased in *M. pneumoniae* infections, which added to their diagnostic performance. This was contrasted by HNL dimer that was lower in *M*. *pneumoniae* as compared to bacterial infections. However, in the logistic regression analysis both biomarkers added significantly to the diagnostic performance resulting in a very high AUC (0.95) and a percent correctly classified patient of 88%. The ratio between TK1 and P-HNL dimer seemed to be the superior diagnostic ratio for the discrimination between *M. pneumoniae* and bacterial infections and in line with the results of the logistic regression.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible.

### REFERENCES

[1] Gronowitz et al., Application of an in vitro assay for serum thymidine kinase: results on viral disease and malignancies in humans. Int J Cancer. 1984, 33:5-12
[2] Svobodova et al., Prognostic importance of Thymidine kinase in colorectal and breast cancer. Anticancer Research 2007, 27:1907-1910
[3] Topolcan & Holubec, The role of thymidine kinase in cancer disease. Expert Opin Med. Diagn. 2008, 2:129-141
[4] Kumar et al., A clinical evaluation of the TK 210 ELISA in sera from breast cancer patients demonstrates high sensitivity and specificity in all stages of disease. Tumour Biol 2016, 37:11937-11945
[5] Venge et al., Human Neutrophil Lipocalin as a Superior Diagnostic Means To Distinguish between Acute Bacterial and Viral infections. Clin Vaccine Immunol. 2015, 22:1025-1032
[6] AroCell TK 210 ELISA, Thymidine Kinase 1 (TK1), Enzyme Linked Immuno Sorbet assay, Instructions for Use, 31-11TF710B1-04 issued date 2017-05-19, www.e-labeling.eu/ARO1001-15-4

## Claims

1. A method of predicting presence of *Mycoplasma* pneumonia in a subject suffering from a respiratory infection, the method comprising:
determining a level of serum thymidine kinase 1 (STK1) material in a serum or plasma sample obtained from the subject with an anti-TK1 antibody or a fragment thereof; and
estimating a likelihood that the respiratory infection is *Mycoplasma* pneumonia caused by *Mycoplasma pneumoniae* based on the determined level of STK1 material in the serum or plasma sample.

2. The method according to claim 1, further comprising comparing the determined level of STK1 material in the serum or plasma sample with a threshold value, wherein estimating the likelihood comprises estimating the likelihood that the respiratory infection is *Mycoplasma* pneumonia caused by *M. pneumoniae* based on the comparison.

3. The method according to claim 2, wherein estimating the likelihood comprises estimating a high likelihood that the respiratory infection is *Mycoplasma* pneumonia caused by *M*. *pneumoniae* if the determined level of STK1 material in the serum or plasma sample exceeds the threshold value and otherwise estimating a low likelihood that the respiratory infection is *Mycoplasma* pneumonia caused by *M. pneumoniae.*

4. The method according to any of the claims 1 to 3, further comprising:
determining a level of at least one additional biomarker in the serum or plasma sample or another body sample obtained from the subject, wherein the at least one additional biomarker is selected from the group consisting of C-reactive protein (CRP), procalcitonin (PCT), calprotectin and human neutrophil lipocalin (HNL), preferably the group consisting of CRP, PCT and HNL, and more preferably HNL, wherein
estimating the likelihood comprises the likelihood that the respiratory infection is *Mycoplasma* pneumonia caused by *Mycoplasma pneumoniae* based on the determined level of STK1 material in the serum or plasma sample and based on the determined level of the at least one additional biomarker in the serum or plasma sample or the another body sample.

5. A method of classifying a respiratory infection in a subject, the method comprising:
determining a level of serum thymidine kinase 1 (STK1) material in a serum or plasma sample obtained from the subject with an anti-TK1 antibody or a fragment thereof;
classifying the respiratory infection as being a *Mycoplasma* pneumonia caused by *Mycoplasma pneumoniae* if the determined level of STK1 material in the serum or plasma sample exceeds a threshold value and otherwise classifying the respiratory infection as being a viral pneumonia caused by a virus or a bacterial pneumonia caused by a bacterium other than *M. pneumoniae.*

6. The method according to claim 5, wherein classifying the respiratory infection comprises classifying the respiratory infection as being a *Mycoplasma* pneumonia caused by *M*. *pneumoniae* if the determined level of STK1 material in the serum or plasma sample exceeds the threshold value and otherwise classifying the respiratory infection as being a bacterial pneumonia caused by the bacterium other than *M. pneumoniae.*

7. The method according to any of the claims 1 to 6, wherein determining the level of STK1 material comprises:
contacting the serum or plasma sample with the anti-TK1 antibody or the fragment thereof;
determining an amount of anti-TK1 antibody or fragment bound to the STK1 material; and
determining the level of STK1 material in the serum or plasma sample based on the determined amount of bound anti-TK1 antibody or fragment and a standard correlation between an amount of bound anti-TK1 antibody or fragment and a level of STK1 material.

8. The method according to any of the claims 1 to 7, wherein the anti-TK1 antibody has specificity for an epitope consisting of an amino acid sequence from a C-terminal region of TK1, the amino acid sequence is preferably selected from a portion of TK1 ranging from amino acid position 200 to amino acid position 234 in TK1, more preferably ranging from amino acid position 205 to amino acid position 230 in TK1, even more preferably ranging from amino acid position 210 to amino acid position 225 in TK1, and most preferably the epitope is selected from the group consisting of GEAVAARKLF SEQ ID NO: 1, NCPVPGKPGE SEQ ID NO: 2, PVPGKPGEAV SEQ ID NO: 3; and NCPVPGKPGEAV SEQ ID NO: 4.

9. The method according to claim 8, wherein the anti-TK1 antibody is a monoclonal anti-TK1 antibody selected from the group consisting of:
a monoclonal anti-TK1 antibody having specificity for the epitope consisting of GEAVAARKLF SEQ ID NO: 1 and having
a variable heavy (VH) domain complementarity determining region 1 (CDR1) having amino acid sequence DYEMH SEQ ID NO: 5;
a VH domain CDR2 having amino acid sequence AIHPGYGGTAYNQKFKG SEQ ID NO: 6;
a VH domain CDR3 having amino acid sequence FITKFDY SEQ ID NO: 7;
a variable light (VL) domain CDR1 having amino acid sequence KSSQSLLDSDGKTFLN SEQ ID NO: 8;
a VL domain CDR2 having amino acid sequence LVSKLDS SEQ ID NO: 9; and
a VL domain CDR3 having amino acid sequence WQGTHFPWT SEQ ID NO: 10;
a monoclonal anti-TK1 antibody having specificity for the epitopes NCPVPGKPGE SEQ ID NO: 2, PVPGKPGEAV SEQ ID NO: 3 and NCPVPGKPGEAV SEQ ID NO: 4 and having
a variable heavy (VH) domain complementarity determining region 1 (CDR1) having amino acid sequence DYEMH SEQ ID NO: 5;
a VH domain CDR2 having amino acid sequence AILPGSGGTAYNQKFKG SEQ ID NO: 17;
a VH domain CDR3 having amino acid sequence LITTFDY SEQ ID NO: 18;
a variable light (VL) domain CDR1 having amino acid sequence KSSQSLLDSDGKTYLN SEQ ID NO: 19;
a VL domain CDR2 having amino acid sequence LVSKLDS SEQ ID NO: 9; and
a VL domain CDR3 having amino acid sequence WQGTHFPWT SEQ ID NO: 10; and
a monoclonal anti-TK1 antibody having specificity for a conformation dependent epitope of TK1 and having
a variable heavy (VH) domain complementarity determining region 1 (CDR1) having amino acid sequence SGYSWH SEQ ID NO: 11;
a VH domain CDR2 having amino acid sequence YIHYSGSTTYNPSLKG SEQ ID NO: 12;
a VH domain CDR3 having amino acid sequence WGTGHWYFDV SEQ ID NO: 13;
a variable light (VL) domain CDR1 having amino acid sequence RSSTGAVTTTNYAN SEQ ID NO: 14;
a VL domain CDR2 having amino acid sequence GTNNRVP SEQ ID NO: 15; and
a VL domain CDR3 having amino acid sequence ALWYSNHWV SEQ ID NO: 16.

10. The method according to any of the claims 1 to 9, wherein determining the level of STK1 material comprises determining the level of STK1 material in the serum or plasma sample obtained from the subject with a kit, preferably an enzyme-linked immunosorbent assay (ELISA) kit, and more preferably a sandwich ELISA kit, comprising:
a first anti-TK1 antibody immobilized to a support or intended to be immobilized to the support; and
a second anti-TK1 antibody, wherein the first and second anti-TK1 antibodies are preferably selected among the monoclonal anti-TK1 antibodies as defined in claim 9.

## Patentansprüche

1. Verfahren zur Prognose des Vorliegens von Mykoplasmen-Pneumonie bei einem Subjekt, das an einer Atemwegsinfektion leidet, wobei das Verfahren Folgendes umfasst:
Bestimmen einer Konzentration von Serum-Thymidinkinase 1-(SKT1-)Material in einer Serum- oder Plasmaprobe, die von dem Subjekt erhalten wurde, mit einem Anti-TK1-Antikörper oder einem Fragment davon; und
Schätzen einer Wahrscheinlichkeit, dass die Atemwegsinfektion Mykoplasmen-Pneumonie ist, die durch Mycoplasma pneumoniae verursacht wurde, auf Grundlage der bestimmen Konzentration von STK1-Material in der Serum- oder Plasmaprobe.

2. Verfahren nach Anspruch 1, ferner umfassend Vergleichen der bestimmten Konzentration von STK1-Material in der Serum- oder Plasmaprobe mit einem Schwellenwert, wobei das Schätzen der Wahrscheinlichkeit Schätzen der Wahrscheinlichkeit, dass die Atemwegsinfektion Mykoplasmen-Pneumonie ist, die durch M. pneumoniae verursacht wurde, auf Grundlage des Vergleichs umfasst.

3. Verfahren nach Anspruch 2, wobei das Schätzen der Wahrscheinlichkeit Schätzen einer hohen Wahrscheinlichkeit, dass die Atemwegsinfektion Mykoplasmen-Pneumonie ist, die durch M. pneumoniae verursacht wurde, wenn die bestimmte Konzentration von STK1-Material in der Serum- oder Plasmaprobe den Schwellenwert überschreitet, und andernfalls Schätzen einer geringen Wahrscheinlichkeit, dass die Atemwegsinfektion Mykoplasmen-Pneumonie ist, die durch M. pneumoniae verursacht wurde, umfasst.

4. Verfahren nach einem der Ansprüche 1 bis 3, ferner umfassend:
Bestimmen einer Konzentration mindestens eines zusätzlichen Biomarkers in der Serum- oder Plasmaprobe oder einer anderen Körperprobe, die von dem Subjekt erhalten wurde, wobei der mindestens eine zusätzliche Biomarker ausgewählt ist aus der Gruppe, bestehend aus C-reaktivem Protein (CRP), Procalcitonin (PCT), Calprotectin und humanem neutrophilen Lipocalin (HNL), bevorzugt aus der Gruppe, bestehend aus CRP, PCT und HNL, und stärker bevorzugt HNL, wobei
das Schätzen der Wahrscheinlichkeit die Wahrscheinlichkeit, dass die Atemwegsinfektion Mykoplasmen-Pneumonie ist, die durch Mycoplasma pneumoniae verursacht wurde, auf Grundlage der bestimmten Konzentration von STK1-Material in der Serum- oder Plasmaprobe und auf Grundlage der bestimmten Konzentration des mindestens einen zusätzlichen Biomarkers in der Serum- oder Plasmaprobe oder der anderen Körperprobe umfasst.

5. Verfahren zum Klassifizieren einer Atemwegsinfektion bei einem Subjekt, wobei das Verfahren Folgendes umfasst:
Bestimmen einer Konzentration von Serum-Thymidinkinase 1-(SKT1-)Material in einer Serum- oder Plasmaprobe, die von dem Subjekt erhalten wurde, mit einem Anti-TK1-Antikörper oder einem Fragment davon;
Klassifizieren der Atemwegsinfektion als Mykoplasmen-Pneumonie, die durch Mycoplasma pneumoniae verursacht wurde, wenn die bestimmte Konzentration von STK1-Material in der Serum- oder Plasmaprobe einen Schwellenwert überschreitet, und anderenfalls Klassifizieren der Atemwegsinfektion als virale Pneumonie, die durch einen Virus verursacht wurde, oder als bakterielle Pneumonie, die durch ein anderes Bakterium als M. pneumoniae verursacht wurde.

6. Verfahren nach Anspruch 5, wobei das Klassifizieren der Atemwegsinfektion Klassifizieren der Atemwegsinfektion als Mykoplasmen-Pneumonie, die durch M. pneumoniae verursacht wurde, wenn die bestimmte Konzentration von STK1-Material in der Serum- oder Plasmaprobe den Schwellenwert überschreitet, und anderenfalls Klassifizieren der Atemwegsinfektion als bakterielle Pneumonie, die durch ein anderes Bakterium als M. pneumoniae verursacht wurde, umfasst.

7. Verfahren nach einem der Ansprüche 1 bis 6, wobei das Bestimmen der Konzentration von STK1-Material Folgendes umfasst:
Inkontaktbringen der Serum- oder Plasmaprobe mit dem Anti-TK1-Antikörper oder dem Fragment davon;
Bestimmen einer Menge des an das STK1-Material gebundenen Anti-TK1-Antikörpers oder Fragments; und
Bestimmen der Konzentration von STK1-Material in der Serum- oder Plasmaprobe auf Grundlage der bestimmten Menge des gebundenen Anti-TK1-Antikörpers oder Fragments und einer Standardkorrelation zwischen einer Menge des gebundenen Anti-TK1-Antikörpers oder Fragments und einer Konzentration von STK1-Material.

8. Verfahren nach einem der Ansprüche 1 bis 7, wobei der Anti-TK1-Antikörper Spezifität für ein Epitop aufweist, das aus einer Aminosäuresequenz von einer C-terminalen Region von TK1 besteht, die Aminosäuresequenz bevorzugt ausgewählt ist aus einem Abschnitt von TK1, der von Aminosäureposition 200 bis Aminosäureposition 234 in TK1 reicht, stärker bevorzugt von Aminosäureposition 205 bis Aminosäureposition 230 in TK1 reicht, noch stärker bevorzugt von Aminosäureposition 210 bis Aminosäureposition 225 in TK1 reicht, und am stärksten bevorzugt das Epitop ausgewählt ist aus der Gruppe, bestehend aus GEAVAARKLF SEQ ID NO: 1, NCPVPGKPGE SEQ ID NO: 2, PVPGKPGEAV SEQ ID NO: 3 und NCPVPGKPGEAV SEQ ID NO: 4.

9. Verfahren nach Anspruch 8, wobei der Anti-TK1-Antikörper ein monoklonaler Anti-TK1-Antikörper ist, der ausgewählt ist aus der Gruppe, bestehend aus:
einem monoklonalen Anti-TK1-Antikörper, der Spezifität für das Epitop, bestehend aus GEAVAARKLF SEQ ID NO: 1, aufweist und Folgendes aufweist:
eine komplementaritätsbestimmende Region 1 (CDR1) der variablen Domäne der schweren Kette (VH-Domäne), die Aminosäuresequenz DYEMH SEQ ID NO: 5 aufweist;
eine VH-Domäne-CDR2, die Aminosäuresequenz AIHPGYGGTAYNQKFKG SEQ ID NO: 6 aufweist;
eine VH-Domäne-CDR3, die Aminosäuresequenz FITKFDY SEQ ID NO: 7 aufweist;
eine CDR1 der variablen Domäne der leichten Kette (VL-Domäne), die Aminosäuresequenz KSSQSLLDSDGKTFLN SEQ ID NO: 8 aufweist;
eine VL-Domäne-CDR2, die Aminosäuresequenz LVSKLDS SEQ ID NO: 9 aufweist; und
eine VL-Domäne-CDR3, die Aminosäuresequenz WQGTHFPWT SEQ ID NO: 10 aufweist;
einem monoklonalen Anti-TK1-Antikörper, der Spezifität für die Epitope NCPVPGKPGE SEQ ID NO: 2, PVPGKPGEAV SEQ ID NO: 3 und NCPVPGKPGEAV SEQ ID NO: 4 aufweist und Folgendes aufweist:
eine komplementaritätsbestimmende Region 1 (CDR1) der variablen Domäne der schweren Kette (VH-Domäne), die Aminosäuresequenz DYEMH SEQ ID NO: 5 aufweist;
eine VH-Domäne-CDR2, die Aminosäuresequenz AILPGSGGTAYNQKFKG SEQ ID NO: 17 aufweist;
eine VH-Domäne-CDR3, die Aminosäuresequenz LITTFDY SEQ ID NO: 18 aufweist;
eine CDR1 der variablen Domäne der leichten Kette (VL-Domäne), die Aminosäuresequenz KSSQSLLDSDGKTYLN SEQ ID NO: 19 aufweist;
eine VL-Domäne-CDR2, die Aminosäuresequenz LVSKLDS SEQ ID NO: 9 aufweist; und
eine VL-Domäne-CDR3, die Aminosäuresequenz WQGTHFPWT SEQ ID NO: 10 aufweist; und
einem monoklonalen Anti-TK1-Antikörper, der Spezifität für ein konformationsabhängiges Epitop von TK1 aufweist und Folgendes aufweist:
eine komplementaritätsbestimmende Region 1 (CDR1) der variablen Domäne der schweren Kette (VH-Domäne), die Aminosäuresequenz SGYSWH SEQ ID NO: 11 aufweist;
eine VH-Domäne-CDR2, die Aminosäuresequenz YIHYSGSTTYNPSLKG SEQ ID NO: 12 aufweist;
eine VH-Domäne-CDR3, die Aminosäuresequenz WGTGHWYFDV SEQ ID NO: 13 aufweist;
eine CDR1 der variablen Domäne der leichten Kette (VL-Domäne), die Aminosäuresequenz RSSTGAVTTTNYAN SEQ ID NO: 14 aufweist;
eine VL-Domäne-CDR2, die Aminosäuresequenz GTNNRVP SEQ ID NO: 15 aufweist; und
eine VL-Domäne-CDR3, die Aminosäuresequenz ALWYSNHWV SEQ ID NO: 16 aufweist.

10. Verfahren nach einem der Ansprüche 1 bis 9, wobei das Bestimmen der Konzentration von STK1-Material Bestimmen der Konzentration von STK1-Material in der Serum- oder Plasmaprobe, die von dem Subjekt erhalten wurde, mit einem Kit, bevorzugt einem Enzyme-linked Immunosorbent Assay (ELISA) und stärker bevorzugt mit einem Sandwich-ELISA-Kit, umfasst, das Folgendes umfasst:
einen ersten Anti-TK1-Antikörper, der auf einem Träger immobilisiert ist oder dafür vorgesehen ist, auf dem Träger immobilisiert zu werden, und
einen zweiten Anti-TK1-Antikörper, wobei der erste und der zweite Anti-TK1-Antikörper bevorzugt aus den monoklonalen Anti-TK1-Antikörpern wie in Anspruch 9 definiert ausgewählt sind.

## Revendications

1. Procédé de prédiction de la présence d'une pneumonie à *Mycoplasma* chez un sujet souffrant d'une infection respiratoire, le procédé comprenant :
la détermination d'un niveau de matière de thymidine kinase 1 sérique (STK1) dans un échantillon de sérum ou de plasma obtenu auprès du sujet à l'aide d'un anticorps anti-TK1 ou d'un fragment de celui-ci ; et
l'estimation de la probabilité que l'infection respiratoire soit une pneumonie à *Mycoplasma* provoquée par *Mycoplasma pneumoniae* sur la base du niveau déterminé de matière STK1 dans l'échantillon de sérum ou de plasma.

2. Procédé selon la revendication 1, comprenant en outre la comparaison du niveau déterminé de matière STK1 dans l'échantillon de sérum ou de plasma avec une valeur seuil, dans lequel l'estimation de la probabilité comprend l'estimation de la probabilité que l'infection respiratoire soit une pneumonie à *Mycoplasma* provoquée par *M. pneumoniae* sur la base de la comparaison.

3. Procédé selon la revendication 2, dans lequel l'estimation de la probabilité comprend l'estimation d'une forte probabilité que l'infection respiratoire soit une pneumonie à *Mycoplasma* provoquée par *M. pneumoniae* si le niveau déterminé de matière STK1 dans l'échantillon de sérum ou de plasma dépasse la valeur seuil et, dans le cas contraire, l'estimation d'une faible probabilité que l'infection respiratoire soit une pneumonie à *Mycoplasma* provoquée par *M. pneumoniae.*

4. Procédé selon l'une quelconque des revendications 1 à 3, comprenant en outre :
la détermination d'un niveau d'au moins un biomarqueur supplémentaire dans l'échantillon de sérum ou de plasma ou dans un autre échantillon corporel obtenu auprès du sujet, dans lequel l'au moins un biomarqueur supplémentaire est choisi dans le groupe constitué de la protéine C-réactive (CRP), de la procalcitonine (PCT), de la calprotectine et de la lipocaline neutrophile humaine (HNL), de préférence le groupe constitué de CRP, PCT et HNL, et de manière davantage préférée HNL, dans lequel
l'estimation de la probabilité comprend la probabilité que l'infection respiratoire soit une pneumonie à *Mycoplasma* provoquée par *Mycoplasma pneumoniae* sur la base du niveau déterminé de matière STK1 dans l'échantillon de sérum ou de plasma et sur la base du niveau déterminé de l'au moins un biomarqueur supplémentaire dans l'échantillon de sérum ou de plasma ou dans l'autre échantillon corporel.

5. Procédé de classification d'une infection respiratoire chez un sujet, le procédé comprenant :
la détermination d'un niveau de matière de thymidine kinase 1 sérique (STK1) dans un échantillon de sérum ou de plasma obtenu auprès du sujet à l'aide d'un anticorps anti-TK1 ou d'un fragment de celui-ci ;
la classification de l'infection respiratoire comme étant une pneumonie à *Mycoplasma* provoquée par *Mycoplasma pneumoniae* si le niveau déterminé de matière STK1 dans l'échantillon de sérum ou de plasma dépasse une valeur seuil et, dans le cas contraire, la classification de l'infection respiratoire comme étant une pneumonie virale provoquée par un virus ou une pneumonie bactérienne provoquée par une bactérie autre que *M. pneumoniae.*

6. Procédé selon la revendication 5, dans lequel la classification de l'infection respiratoire comprend la classification de l'infection respiratoire comme étant une pneumonie à *Mycoplasma* provoquée par *M. pneumoniae* si le niveau déterminé de matière STK1 dans l'échantillon de sérum ou de plasma dépasse la valeur seuil et, dans le cas contraire, la classification de l'infection respiratoire comme étant une pneumonie bactérienne provoquée par la bactérie autre que *M. pneumoniae.*

7. Procédé selon l'une quelconque des revendications 1 à 6, dans lequel la détermination du niveau de matière STK1 comprend :
la mise en contact de l'échantillon de sérum ou de plasma avec l'anticorps anti-TK1 ou le fragment de celui-ci ;
la détermination d'une quantité d'anticorps ou de fragment anti-TK1 lié à la matière STK1 ; et
la détermination du niveau de matière STK1 dans l'échantillon de sérum ou de plasma sur la base de la quantité déterminée d'anticorps ou de fragment anti-TK1 lié et d'une corrélation standard entre une quantité d'anticorps ou de fragment anti-TK1 lié et un niveau de matière STK1.

8. Procédé selon l'une quelconque des revendications 1 à 7, dans lequel l'anticorps anti-TK1 a une spécificité pour un épitope constitué d'une séquence d'acides aminés provenant d'une région C-terminale de TK1, la séquence d'acides aminés est de préférence choisie parmi une partie de TK1 allant de la position d'acide aminé 200 à la position d'acide aminé 234 dans TK1, de manière davantage préférée allant de la position d'acide aminé 205 à la position d'acide aminé 230 dans TK1, de manière encore davantage préférée allant de la position d'acide aminé 210 à la position d'acide aminé 225 dans TK1, et de manière préférée entre toutes, l'épitope est choisi dans le groupe constitué de GEAVAARKLF SEQ ID NO : 1, NCPVPGKPGE SEQ ID NO : 2, PVPGKPGEAV SEQ ID NO : 3 ; et NCPVPGKPGEAV SEQ ID NO : 4.

9. Procédé selon la revendication 8, dans lequel l'anticorps anti-TK1 est un anticorps monoclonal anti-TK1 choisi dans le groupe constitué de :
un anticorps monoclonal anti-TK1 ayant une spécificité pour l'épitope constitué de GEAVAARKLF SEQ ID NO : 1 et ayant
une région déterminant la complémentarité 1 (CDR1) de domaine lourd variable (VH) ayant la séquence d'acides aminés DYEMH SEQ ID NO : 5 ;
une CDR2 de domaine VH ayant la séquence d'acides aminés AIHPGYGGTAYNQKFKG SEQ ID NO : 6 ;
une CDR3 de domaine VH ayant la séquence d'acides aminés FITKFDY SEQ ID NO : 7 ;
une CDR1 de domaine léger variable (VL) ayant la séquence d'acides aminés KSSQSLLDSDGKTFLN SEQ ID NO : 8 ;
une CDR2 de domaine VL ayant la séquence d'acides aminés LVSKLDS SEQ ID NO : 9 ; et
une CDR3 de domaine VL ayant la séquence d'acides aminés WQGTHFPWT SEQ ID NO : 10 ;
un anticorps monoclonal anti-TK1 ayant une spécificité pour les épitopes NCPVPGKPGE SEQ ID NO : 2, PVPGKPGEAV SEQ ID NO : 3 et NCPVPGKPGEAV SEQ ID NO : 4 et ayant
une région déterminant la complémentarité 1 (CDR1) de domaine lourd variable (VH) ayant la séquence d'acides aminés DYEMH SEQ ID NO : 5 ;
une CDR2 de domaine VH ayant la séquence d'acides aminés AILPGSGGTAYNQKFKG SEQ ID NO : 17 ;
une CDR3 de domaine VH ayant la séquence d'acides aminés LITTFDY SEQ ID NO : 18 ;
une CDR1 de domaine léger variable (VL) ayant la séquence d'acides aminés KSSQSLLDSDGKTYLN SEQ ID NO : 19 ;
une CDR2 de domaine VL ayant la séquence d'acides aminés LVSKLDS SEQ ID NO : 9 ; et
une CDR3 de domaine VL ayant la séquence d'acides aminés WQGTHFPWT SEQ ID NO : 10 ; et
un anticorps monoclonal anti-TK1 ayant une spécificité pour un épitope dépendant de la conformation de TK1 et ayant
une région déterminant la complémentarité 1 (CDR1) de domaine lourd variable (VH) ayant la séquence d'acides aminés SGYSWH SEQ ID NO : 11 ;
une CDR2 de domaine VH ayant la séquence d'acides aminés YIHYSGSTTYNPSLKG SEQ ID NO : 12 ;
une CDR3 de domaine VH ayant la séquence d'acides aminés WGTGHWYFDV SEQ ID NO : 13 ;
une CDR1 de domaine léger variable (VL) ayant la séquence d'acides aminés RSSTGAVTTTNYAN SEQ ID NO : 14 ;
une CDR2 de domaine VL ayant la séquence d'acides aminés GTNNRVP SEQ ID NO : 15 ; et
une CDR3 de domaine VL ayant la séquence d'acides aminés ALWYSNHWV SEQ ID NO : 16.

10. Procédé selon l'une quelconque des revendications 1 à 9, dans lequel la détermination du niveau de matière STK1 comprend la détermination du niveau de matière STK1 dans l'échantillon de sérum ou de plasma obtenu auprès du sujet avec un kit, de préférence un kit de dosage immunosorbant lié aux enzymes (ELISA), et de manière davantage préférée un kit ELISA sandwich, comprenant :
un premier anticorps anti-TK1 immobilisé sur un support ou destiné à être immobilisé sur le support ; et
un second anticorps anti-TK1, dans lequel les premier et second anticorps anti-TK1 sont de préférence choisis parmi les anticorps monoclonaux anti-TK1 tels que définis dans la revendication 9.
